# EUROPEAN PATENT APPLICATION

(11) **EP 1 420 005 A1**
(43) Date of publication of application: **19.05.2004**
(21) Application number: 02755670.3
(22) Date of filing: 29.07.2002
(51) Int. Cl.: C07C 35/21, C07C 29/143, C07C 29/17, C07C 49/417

(54) **CYCLOPENTANONE DERIVATIVE**

(30) Priority: 30.07.2001 JP 2001228891
(71) Applicant: Zeon Corporation, Tokyo 100-8323 (JP)
(72) Inventor: FUJISAWA, Hiroshi, c/o ZEON CORPORATION, Tokyo 100-8323 (JP); KONDOU, Yoshihisa, c/o ZEON CORPORATION, Tokyo 100-8323 (JP)
(74) Representative: Albrecht, Thomas, Dr.
(86) International application number: PCT/JP2002/007638
(87) International publication number: WO 2003/011803

(57) **Abstract**

A novel cyclopentanone derivative having a cyclopentanone or cyclopentanol ring having substituents at the 2- and 5-positions is provided. The cyclopentanone derivative preferably has 13 to 17 carbon atoms. A perfume composition comprising the cyclopentanone derivative emits a flora fragrance emphasizing a natural and fresh feeling. It is useful for perfuming a variety of toiletries and households. A process for preparing the above-mentioned cyclopentanone derivative is also provided by which the cyclopentanone derivative can be obtained in a practically acceptable yield.

## Description

### TECHNICAL FIELD

This invention relates to a cyclopentanone derivative and a perfume composition comprising the same, which are used for a perfume, a soap, a shampoo, a hair rinse, a body shampoo, a detergent, a cosmetic, a hair spray, an aromatic, and others.

### BACKGROUND ART

It is known that cyclopentanone derivatives include those which are useful as a perfume. As specific examples of the cyclopentanone derivatives used as a perfume, there can be mentioned methyl 2-(cis-2-pentenyl)-3-oxocyclopent-3-yl-acetate (trivial name: methyl jasmonate) and methyl 2-n-pentenyl-3-oxocyclopent-3-yl-acetate (trivial name: methyl dihydrojasmonate), which are known as a perfume emitting a jasmin-like floral scent; and 2-cyclopentyl cyclopentylcrotonate which is known as a perfume emitting a fruity and juicy scent.

A cyclopentanone derivative represented by the following general formula (A): 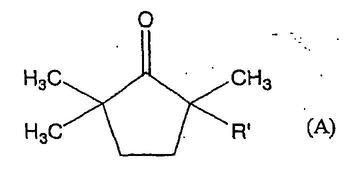 wherein R^{L} represents an alkyl group having 4 to 6 carbon atoms, and a fragrance-emitting or flavor-giving preparation comprising the cyclopentanone 'derivative have been proposed in Japanese Unexamined Patent Publication No. S52-139046. It is described in this publication that this cyclopentanone derivative emits a fruity and fresh apricot-like fragrance.

User's or consumer's fancy for fragrance-emitting preparations and products varies depending upon the particular age and sex distinction. The kinds of such products and the purposes of use thereof are being rapidly expanded, and therefore, various fragrance-emitting or modifying ingredients giving products emitting fragrant scents, which are delicately different in primary tone, depth, amplitude and volume, are desired.

A perfume ingredient is a kind of physiologically active substance. It is known that modification of its chemical structure delicately influences and occasionally greatly changes the fragrance of the ingredient, perceived by a human being. Therefore it is important for developing a novel perfume compound to synthesize analogues and derivatives of a known perfume compound and assess the fragrance thereof.

### DISCLOSURE OF THE INVENTION

An object of the present invention is to provide a cyclopentanone derivative useful as a novel perfume ingredient emitting a floral fragrance, and to provide a perfume composition comprising the cyclopentanone derivative.

To achieve the above-mentioned object, the present inventors synthesized various cyclopentanone derivatives having substituents at 2- and 5-positions of a cyclopentanone or cyclopentanol structure, and assessed their fragrances and perfume compositions comprising the same. Consequently, the present inventors have found that specific cyclopentanone derivatives emit a floral fragrance, and that perfume compositions comprising the cyclopentanone derivatives diffuse a floral fragrance and are useful for giving a natural and fresh scent to variety of toiletries. Based on these findings, the present invention has been completed.

Another object of the present invention is to provide a process for preparing the above-mentioned cyclopentanone derivatives in a practically acceptable yield.

Thus, in accordance with the present invention, there are provided the following cyclopentanone derivatives and perfume compositions.
(i) A cyclopentanone derivative represented by the following general formula (1): 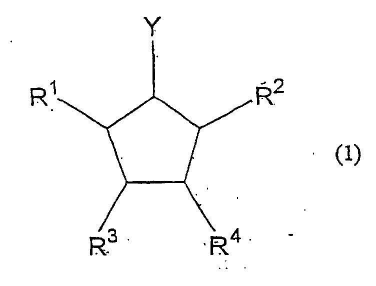 wherein R¹ represents an alkyl group having 4 to 7 carbon atoms, an alkylidene group having 4 to 7 carbon atoms, a cyclohexyl group or a cyclohexylidene group, R² represents an alkyl group having 4 to 7 carbon atoms, an alkylidene group having 4 to 7 carbon atoms, a cyclopentyl group, a cyclopentylidene group, a cyclohexyl group or a cyclohexylidene group, R³ and R⁴ independently represent a hydrogen atom or an alkyl group having 1 to 4 carbon atoms, and -Y represents -OH or =O.
(ii) A cyclopentanone derivative represented by the following general formula (2): 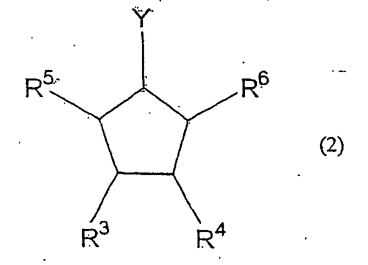 wherein R⁵ represents an alkyl group having 4 or 5 carbon atoms or an alkylidene group having 4 or 5 carbon atoms, R⁶ represents an alkyl group having 4 or 5 carbon atoms, an alkylidene group having 4 or 5 carbon atoms, a cyclopentyl group or a cyclopentylidene group, and R³, R⁴ and -Y are the same as defined above.
(iii) A cyclopentanone derivative represented by the following general formula (3): 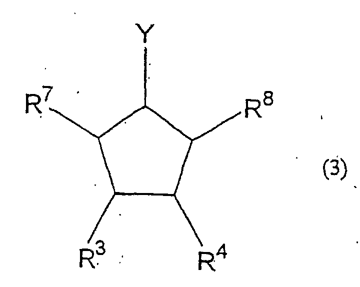 wherein R⁷ represents an alkyl group having 5 carbon atoms, or an alkylidene group having 5 carbon atoms, R⁸ represents an alkyl group having 5 carbon atoms, an alkylidene group having 5 carbon atoms, a cyclopentyl group, or a cyclopentylidene group, and R³, R⁴ and -Y are the same as defined above.
(iv) The cyclopentanone derivative according to any one of the above paragraphs (i) to (iii), which has 13 to 17 carbon atoms in total.
(v) The cyclopentanone derivative according to any one of the above paragraphs (i) to (iv), wherein both of R³ and R⁴ are hydrogen atoms.
(vi) A perfume composition comprising at least one cyclopentanone derivative selected from those which are described in the above paragraphs (i) to (v).
(vii) A perfume composition comprising 0.1 to 90% by weight of a cyclopentanone derivative selected from those which are described in the above paragraphs (i) to (v).
(viii) In accordance with the present invention, there is further provided a process for preparing a cyclopentanone derivative represented by the following general formula (6): 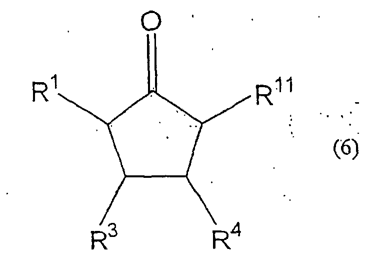 wherein R¹, R³ and R⁴ are the same as defined above, and R¹¹ represents an alkylidene group having 4 to 7 carbon atoms, a cyclopentylidene group or a cyclohexylidene group,
   characterized in that a cyclopentanone derivative represented by the following general formula (4): 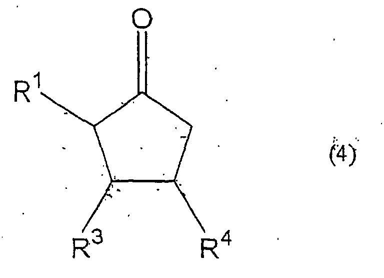 wherein R¹, R³ and R⁴ are the same as defined above, is reacted under alkaline conditions with a ketone or aldehyde represented by the following general formula (5): 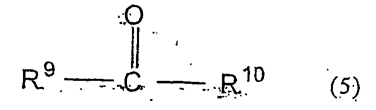 wherein R⁹ and R¹⁰ represents a hydrogen atom, or an alkyl group having 1 to 6 carbon atoms; the alkyl groups for R⁹ and R¹⁰ may be bonded together to form a ring; and the total number of carbon atoms in the sum of the alkyl groups for R⁹ and R¹⁰ is in the range of 3 to 6.
(ix) In accordance with the present invention, there is further provided a process for preparing a cyclopentanone derivative represented by the following general formula (7): 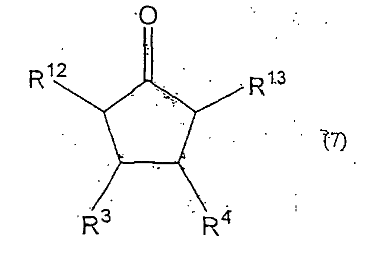 wherein R³ and R⁴ are the same as defined above, R¹² represents an alkyl group having 4 to 7 carbon atoms or a cyclohexyl group, and R¹³ represents an alkyl group having 4 to 7 carbon atoms, a cyclopentyl group or a cyclohexyl group,
   characterized in that a cyclopentanone derivative represented by the above-mentioned general formula (6) is reduced with hydrogen.
(x) In accordance with the present invention, there is further provided a process for preparing a cyclopentanone derivative represented by the following general formula (8): 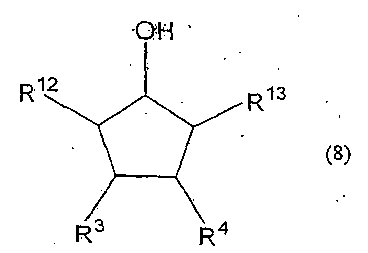 wherein R³, R⁴, R¹² and R¹³ are the same as defined above,
   characterized in that a cyclopentanone derivative represented by the above-mentioned general formula (7) is reduced with hydrogen.
(xi) In accordance with the present invention, there is further provided a process for preparing a cyclopentanone derivative represented by the above-mentioned general formula (8),
characterized in that a cyclopentanone derivative represented by the following general formula (9): 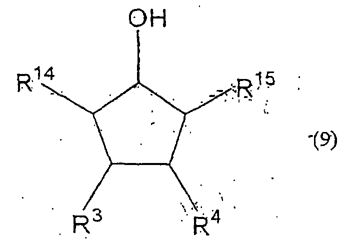 wherein R³ and R⁴ are the same as defined above, R¹⁴ represents an alkyl group having 4 to 7 carbon atoms, an alkylidene group having 4 to 7 carbon atoms, a cyclohexyl group or a cyclohexylidene group, R¹⁵ represents an alkyl group having 4 to 7 carbon atoms, an alkylidene group having 4 to 7 carbon atoms, a cyclopentyl group, a cyclopentylidene group, a cyclohexyl group or a cyclohexylidene group, and at least one of R¹⁴ and R¹⁵ is the alkylidene group, a cyclohexylidene group or a cyclopentylidene group, is reduced with hydrogen.

### BEST MODE FOR CARRYING OUT THE INVENTION

The invention will now be described in detail with respect to cyclopentanone derivatives, processes for preparing the cyclopentanone derivatives and perfume compositions.

### (1) Cyclopentanone deivatives

The present invention is concerned with cyclopentanone derivatives represented by the above-mentioned general formula (1).

In the general formula (1), R¹ includes, for example, chain alkyl groups having 4 to 7 carbon atoms, such as n-butyl group, isobutyl group, s-butyl group, t-butyl group, n-pentyl group, neopentyl group, isopentyl group, t-pentyl group, n-hexyl group, isohexyl group and n-heptyl group; chain alkylidene groups having 4 to 7 carbon atoms, such as n-butylidene group, isobutylidene group, n-pentylidene group, neopentylidene group, isopentylidene group, n-hexylidene group, isohexylidene group and n-heptylidene group; and a cyclohexyl group and a cyclohexylidene group.

R² includes, for example, chain alkyl groups having 4 to 7 carbon atoms, such as n-butyl group, isobutyl group, s-butyl group, t-butyl group, n-pentyl group, neopentyl group, isopentyl group, t-pentyl group, n-hexyl group, isohexyl group and n-heptyl group; chain alkylidene groups having 4 to 7 carbon atoms, such as n-butylidene group, isobutylidene group, n-pentylidene group, neopentylidene group, isopentylidene group, n-hexylidene group, isohexylidene group and n-heptylidene group; and a cyclopentyl group, cyclopentylidene group, a cyclohexyl group and a cyclohexylidene group.

R³ and R⁴ independently represent a hydrogen atom, or an alkyl group having 1 to 4 carbon atoms, which include, for example, methyl, ethyl, n-propyl, isopropyl and n-butyl groups.

-Y represents -OH or =O.

The cyclopentanone derivative represented by the general formula (1) preferably has 13 to 17 carbon atoms in total. As specific examples thereof, there can be mentioned the following cyclopentanone derivatives.
(1-1) Cyclopentanone derivatives wherein -Y is =O 2,5-di-n-butylidenecyclopentanone, 2,5-diisobutylidenecyclopentanone, 2,5-di-n-pentylidenecyclopentanone, 2,5-dineopentylidenecyclopentanone, 2,5-diisopentylidenecyclopentanone, 2,5-di-n-hexylidenecyclopentanone, 2,5-diisohexylidenecyclopentanone, 2,5-dicyclohexylidenecyclopentanone, 2-n-butylidene-5-isobutylidenecyclopentanone, 2-n-butylidene-5-n-pentylidenecyclopentanone, 2-n-butylidene-5-neopentylidenecyclopentanone, 2-n-butylidene-5-isopentylidenecyclopentanone, 2-n-butylidene-5-n-hexylidenecyclopentanone, 2-n-butylidene-5-isohexylidenecyclopentanone, 2-n-butylidene-5-n-heptylidenecyclopentanone, 2-n-pentylidene-5-isobutylidenecyclopentanone, 2-n-pentylidene-5-neopentylidenecyclopentanone, 2-n-pentylidene-5-isopentylidenecyclopentanone, 2-n-pentylidene-5-n-hexylidenecyclopentanone, 2-n-pentylidene-5-isohexylidenecyclopentanone, 2-n-pentylidene-5-n-heptylidenecyclopentanone, 2-n-hexylidene-5-isobutylidenecyclopentanone, 2-n-hexylidene-5-neopentylidenecyclopentanone, 2-n-hexylidene-5-isopentylidenecyclopentanone, 2-n-hexylidene-5-isohexylidenecyclopentanone, 2-cyclopentylidene-5-n-butylidenecyclopentanone, 2-cyclopentylidene-5-isobutylidenecyclopentanone, 2-cyclopentylidene-5-n-pentylidenecyclopentanone, 2-cyclopentylidene-5-neopentylidenecyclopentanone, 2-cyclopentylidene-5-isopentylidenecyclopentanone, 2-cyclopentylidene-5-n-hexylidenecyclopentanone, 2-cyclopentylidene-5-isohexylidenecyclopentanone, 2-cyclopentylidene-5-n-heptylidenecyclopentanone, 2-cyclopentylidene-5-cyclohexylidenecyclopentanone, 2-cyclohexylidene-5-n-butylidenecyclopentanone, 2-cyclohexylidene-5-isobutylidenecyclopentanone, 2-cyclohexylidene-5-n-pentylidenecyclopentanone, 2-cyclohexylidene-5-neopentylidenecyclopentanone, 2-cyclohexylidene-5-n-hexylidenecyclopentanone, 2-cyclohexylidene-5-isohexylidenecyclopentanone, 2-n-pentyl-5-n-butylidenecyclopentanone, 2-n-pentyl-5-n-pentylidenecyclopentanone, 2-n-pentyl-5-n-hexylidenecyclopentanone, 2-n-pentyl-5-cyclopentylidenecyclopentanone, 2-n-pentyl-5-cyclohexylidenecyclopentanone, 2-cyclopentyl-5-n-butylidenecyclopentanone, 2-cyclopentyl-5-n-pentylidenecyclopentanone, 2-cyclopentyl-5-n-hexylidenecyclopentanone, 2-cyclopentyl-5-cyclohexylidenecyclopentanone, 2,5-di-n-butylcyclopentanone, 2,5-diisobutylcyclopentanone, 2,5-di-s-butylcyclopentanone, 2,5-di-n-pentylcyclopentanone, 2,5-dineopentylcyalopentanone, 2,5-di-t-pentylcyclopentanone, 2,5-di-n-hexylcyclopentanone, 2,5-diisohexylcyclopentanone, 2,5-dicyclohexylcyclopentanone, 2-n-butyl- 5-isobutylcyclopentanone, 2-n-butyl-5-n-pentylcyclopentanone, 2-n-butyl-5-neopentylcyclopentanone, 2-n-butyl-5-t-pentylcyclopentanone, 2-n-butyl-5-n-hexylcyclopentanone, 2-n-butyl-5-isohexylcyclopentanone, 2-n-butyl-5-n-heptylcyclopentanone, 2-n-pentyl-5-isobutylcyclopentanone, 2-n-pentyl-5-s-butylcyclopentanone, 2-n-pentyl-5-neopentylcyclopentanone, 2-n-pentyl-5-n-hexylcyclopentanone, 2-n-pentyl-5-isohexylcyclopentanone, 2-n-pentyl-5-n-heptylcyclopentanone, 2-n-hexyl-5-isobutylcyclopentanone, 2-n-hexyl-5-s-butylcyclopentanone, 2-n-hexyl- 5-neopentylcyclopentanone, 2-n-hexyl-5-isopentylclopentanone, 2-n-hexyl-5-t-pentylcyclopentanone, 2-n-hexyl-5-isohexylcyclopentanone, 2-cyclopentyl-5-n-butylcyclopentanone, 2-cyclopentyl-5-s-butylcyclopentanone, 2-cyclopentyl-5-isobutylcyclopentanone, 2-cyclopentyl-5-n-pentylcyclopentanone, 2-cyclopentyl-5-neopentylcyclopentanone, 2-cyclopentyl-5-isopentylcyclopentanone, 2-cyclopentyl-5-t-pentylcyclopentanone, 2-cyclopentyl-5-n-hexylcyclopentanone, 2-cyclopentyl-5-isohexylcyclopentanone, 2-cyclopentyl-5-n-heptylcyclopentanone, 2-cyclohexyl-5-n-butylcyclopentanone, 2-cyclohexyl-5-s-butylcyclopentanone, 2-cyclohexyl-5-isobutylcyclopentanone, 2-cyclohexyl-5-n-pentylcyclopentanone, 2-cyclohexyl-5-neopentylcyclopentanone, 2-cyclohexyl-5-t-pentylcyclopentanone, 2-cyclohexyl-5-n-hexylcyclopentanone, 2-cyclohexyl-5-isohexylcyclopentanone and 2-cyclohexyl-5-cyclopentylcyclopentanone.
(1-2) Cyclopentanone derivatives wherein -Y is -OH 2,5-di-n-butylidenecyclopentanol, 2,5-diisobutylidenecyclopentanol, 2,5-di-n-pentylidenecyclopentanol, 2,5-dineopentylidenecyclopentanol, 2,5-diisopentylidenecyclopentanol, 2,5-di-n-hexylidenecyclopentanol, 2,5-diisohexylidenecyclopentanol, 2,5-dicyclohexylidenecyclopentanol, 2-n-butylidene-5-isobutylidenecyclopentanol, 2-n-butylidene-5-n-pentylidenecyclopentanol, 2-n-butylidene-5-neopentylidenecyclopentanol, 2-n-butylidene-5-isopentylidenecyclopentanol, 2-n-butylidene-5-n-hexylidenecyclopentanol, 2-n-butylidene-5-isohexylidenecyclopentanol, 2-n-butylidene-5-n-heptylidenecyclopentanol, 2-n-pentylidene-5-isobutylidenecyclopentanol, 2-n-pentylidene-5-neopentylidenecyclopentanol, 2-n-pentylidene-5-isopentylidenecyclopentanol, 2-n-pentylidene-5-n-hexylidenecyclopentanol, 2-n-pentylidene-5-isohexylidenecyclopentanol, 2-n-pentylidene-5-n-heptylidenecyclopentanol, 2-n-hexylidene-5-isobutylidenecyclopentanol, 2-n-hexylidene-5-neopentylidenecyclopentanol, 2-n-hexylidene-5-isopentylidenecyclopentanol, 2-n-hexylidene-5-isohexylidenecyclopentanol, 2-cyclopentylidene-5-n-butylidenecyclopentanol, 2-cyclopentylidene-5-isobutylidenecyclopentanol, 2-cyclopentylidene-5-n-pentylidenecyclopentanol, 2-cyclopentylidene-5-neopentylidenecyclopentanol, 2-cyclopentylidene-5-isopentylidenecyclopentanol, 2-cyclopentylidene-5-n-hexylidenecyclopentanol, 2-cyclopentylidene-5-isohexylidenecyclopentanol, 2-cyclopentylidene-5-n-heptylidenecyclopentanol, 2-cyclopentylidene-5-cyclohexylidenecyclopentanol, 2-cyclohexylidene-5-n-butylidenecyclopentanol, 2-cyclohexylidene-5-isobutylidenecyclopentanol, 2-cyclohexylidene-5-n-pentylidenecyclopentanol, 2-cyclohexylidene-5-neopentylidenecyclopentanol, 2-cyclohexylidene-5-isopentylidenecyclopentanol, 2-cyclohexylidene-5-n-hexylidenecyclopentanol, 2-cyclohexylidene-5-isohexylidenecyclopentanol, 2-n-pentyl-5-n-butylidenecyclopentanol, 2-n-pentyl-5-n-pentylidenecyclopentanol, 2-n-pentyl-5-n-hexylidenecyclopentanol, 2-n-pentyl-5-cyclopentylidenecyclopentanol, 2-n-pentyl-5-cyclohexylidenecyclopentanol, 2-cyclopentyl-5-n-butylidenecyclopentanol, 2-cyclopentyl-5-n-pentylidenecyclopentanol, 2-cyclopentyl-5-n-hexylidenecyclopentanol, 2-cyclopentyl-5-cyclohexylidenecyclopentanol, 2,5-di-n-butylcyclopentanol, 2,5-diisobutylcyclopentanol, 2,5-di-s-butylcyclopentanol, 2,5-di-n-pentylcyclopentanol, 2,5-dineopentylcyclopentanol, 2,5-diisopentylcyclopentanol, 2,5-di-t-pentylcyclopentanol, 2,5-di-n-hexylcyclopentanol, 2,5-diisohexylcyclopentanol, 2,5-dicyclohexylcyclopentanol, 2-n-butyl-5-isobutylcyclopentanol, 2-n-butyl-5-s-butylcyclopentanol, 2-n-butyl-5-n-pentylcyclopentanol, 2-n-butyl-5-neopentylcyclopentanol, 2-n-butyl-5-isopentylcyclopentanol, 2-n-butyl-5-n-hexylcyclopentanol, 2-n-butyl-5-isohexylcyclopentanol, 2-n-butyl-5-n-heptylcyclopentanol, 2-n-pentyl-5-isobutylcyclopentanol, 2-n-pentyl-5-s-butylcyclopentanol, 2-n-pentyl-5-neopentylcyclopentanol, 2-n-pentyl-5-isopentylcyclopentanol, 2-n-pentyl-5-t-pentylcyclopentanol, 2-n-pentyl-5-n-hexylcyclopentanol, 2-n-pentyl-5-isohexylcyclopentanol, 2-n-pentyl-5-n-heptylcyclopentanol, 2-n-hexyl-5-isobutylcyclopentanol, 2-n-hexyl-5-s-butylcyclopentanol, 2-n-hexyl-5-neopentylcyclopentanol, 2-n-hexyl-5-isopentylclopentanol, 2-n-hexyl-5-t-pentylcyclopentanol, 2-n-hexyl-5-isohexylcyclopentanol, 2-cyclopentyl-5-n-butylcyclopentanol, 2-cyclopentyl-5-s-butylcyclopentanol, 2-cyclopentyl-5-isobutylcyclopentanol, 2-cyclopentyl-5-n-pentylcyclopentanol, 2-cyclopentyl-5-neopentylcyclopentanol, 2-cyclopentyl-5-isopentylcyclopentanol, 2-cyclopentyl-5-t-pentylcyclopentanol, 2-cyclopentyl-5-n-hexylcyclopentanol, 2-cyclopentyl-5-isohexylcyclopentanol, 2-cyclopentyl-5-n-heptylidenecyclopentanol, 2-cyclohexyl-5-n-butylcyclopentanol, 2-cyclohexyl-5-s-butylcyclopentanol, 2-cyclohexyl-5-isobutylcyclopentanol, 2-cyclohexyl-5-n-pentylcyclopentanol, 2-cyclohexyl-5-neopentylcyclopentanol, 2-cyclohexyl-5-isopentylcyclopentanol, 2-cyclohexyl-5-t-pentylcyclopentanol, 2-cyclohexyl-5-n-hexylcyclopentanol, 2-cyclohexyl-5-isohexylcyclopentanol and 2-cyclohexyl-5-cyclopentylcyclopentanol.

Among the cyclopentanone derivatives of the present invention, those which represented by the above-mentioned general formula (2) are preferable. In the general formula (2), R⁵ represents an alkyl group having 4 or 5 carbon atoms or an alkylidene group having 4 or 5 carbon atoms, among the alkyl and alkylidene groups for R¹ in the formula (1). R⁶ represents an alkyl group having 4 or 5 carbon atoms or an alkylidene group having 4 or 5 carbon atoms, among the alkyl and alkylidene groups for R² in the formula (1), or R⁶ represents a cyclopentyl group or a cyclopentylidene group. R³, R⁴ and -Y are the same as defined above.

More preferable cyclopentanone derivatives of the present invention are represented by the above-mentioned general formula (3). In the general formula (3), R⁷ represents an alkyl group having 5 carbon atoms or an alkylidene group having 5 carbon atoms, among the alkyl and alkylidene groups for R¹ in the formula (1). R⁸ represents an alkyl group having 5 carbon atoms or an alkylidene group having 5 carbon atoms, among the alkyl and alkylidene groups for R² in the formula (1), or R⁸ represents a cyclopentyl group or a cyclopentylidene group. R³, R⁴ and -Y are the same as defined above.

In view of ease in synthesis, cyclopentanone derivatives represented by the general formulae (1), (2) and (3) wherein both of R³ and R⁴ are a hydrogen atom are especially preferable.

### (2) Perfume composition

The present invention is further concerned with a perfume composition comprising one kind or more kinds of cyclopentanone derivatives selected from those are represented by the general formulae (1), (2) and (3). The perfume composition of the present invention can be prepared by mixing together predetermined amounts of one kind or more kinds of cyclopentanone derivatives selected from those are represented by the general formulae (1), (2) and (3), and, if desired, other perfume ingredients and solvent ingredients. The amount of the cyclopentanone derivatives of the general formulae (1), (2) and (3) varies depending upon the particular kind of perfume ingredients and the particular kind and intense of fragrance, but, it is preferably in the range of 0.1 to 90% by weight, more preferably 0.5 to 50% by weight, based on the total weight of the perfume composition.

As specific examples of other perfume ingredients used, there can be mentioned acetyl diisoamylene, acetylcedrene, acetaldehyde diethylacetal, anethole, allyl amyl glycolate, allyl heptanoate, allyl caproate, algue absolute, ambrinol, AMBROXAN™, ionone-α, ionone-β, isobornyl acetate, isocamphylcyclohexanol, indole, ethyllinalol, ethylene brassylate, HEDIONE™, eugenol, 11-oxa-16-hexadecanolide, ortho-tert.-butylcyclohexyl acetate, ortho-tert.-butylcyclohexanone, orange oil, chamomile oil, 1-carvone, CALONE™, camphor, gamma-decalactone, caryophyllene, coumarin, CLAIGEON™, clove bud oil, GALAXOLIDE™, geraniol, geranyl acetate, grape fruit oil, geranylnitrile, copaiba balsam, corps pample 10% LIM, diethyl phthalate, citral, 1,8-cineol, cyclamen aldehyde, ciste absolute, citronella oil, citronellol, citronellyl formate, dihydro-myrcenol, diphenyl oxide, civetone, dimethyl anthranilate, dimethylhydroquinone, dimethylbenzylcarbinol acetate, jasmin oil, JASMOPYRAN™, styralyl acetate, spearmint oil, clary sage oil, cedrol, santenal, citronellyl acetate, dimethylbenzylcarbinyl acetate, damascenone, thymol, tetrahydromuguol, terpineol, terpinyl acetate, TONALID™, triethyl citrate, tricyclodecenyl acetate, TRIPLAL™, terpineol, trimethylundecenal, neroli oil, neryl acetate, nopil acetate, pine oil, BACDANOL™, basil oil, BASILEX™, PEARLIDE™, mint oil, patchouli oil, α-pinene, phenylethyl alcohol, PHENOXANOL™, bulgeonal, frutate, plenyl acetate, hei absolute, cis-3-hexenol, hexyl acetate, β-naphthol ethyl ether, cis-3-hexenyl salicylate, benzyl acetate, hexylcinnamic aldehyde, hexyl salicylate, cis-3-hexenyl acetate, cis-3-hexenyl salicylate, peppermint oil, helional, heliotropin, bergamot oil, VERTENEX™, benzyl acetate, benzyl salicylate, borneol, mayol, methyloctyne carbonate, methyl anthranilate, methyl salicylate, methyl dihydrojasmonate, methylionone, menthone, 1-menthol, eucalyptus oil, lime oil, lavandin grosso, labdanum absolute, lavender oil, limonene, linalool, linalyl acetate, LYRAL™, LILIAL™, lemon oil, rose oil, rosemary oil, rosewood Bulgarian and rose Turkish. These perfume ingredients may be used either alone or as a combination of at least two thereof.

The amount of these perfume ingredients is usually in the range of 0.1 to 500 parts by weight, preferably 1 to 200 parts by weight, per one part by weight of the cyclopentanone derivative of the present invention.

When a carrier is used for impregnation with perfume ingredients, a solvent can be appropriately incorporated in the perfume composition of the present invention to enhance the penetration of perfume ingredients into the carrier. The solvent used includes, for example, ethanol, polyhydric alcohols, paraffins, glycol ethers and phthalic acid esters. When water is used to enhance the penetration of perfume ingredient into a carrier, a surface active agent can be added. When the perfume composition is used as an aromatic, a fixative can be incorporated therein to enhance the preservation of fragrance.

The perfume composition of the present invention diffuses a floral fragrance and is useful for giving a natural and fresh scent to variety of toiletries and household products, which include, for example, a perfume, a soap, a shampoo, a hair rinse, a body shampoo, a detergent, a cosmetic, a hair spray and an aromatic.

### (3) Process for preparation of cyclopentanone derivatives

Among the cyclopentanone derivatives of the general formulae (1) to (3), 2,5-dialkylidenecyclopentanone derivatives (1-1) wherein -Y is =O and R¹ and R² are an alkylidene group can be prepared by a process wherein an aldol reaction of a cyclopentanone compound Ra-CO-Rb, and a subsequent dehydration reaction are carried out to give a monoalkylidenecyclopentanone derivative (3-2), and then, aldol reaction of the monoalkylidenecyclopentanone derivative (3-2) with an aldehyde or ketone represented by the formula: Rc-CO-Rd, and a subsequent dehydration reaction are carried out to give a dialkylidenecyclopentanone derivative (1-1) (see the following reaction scheme). 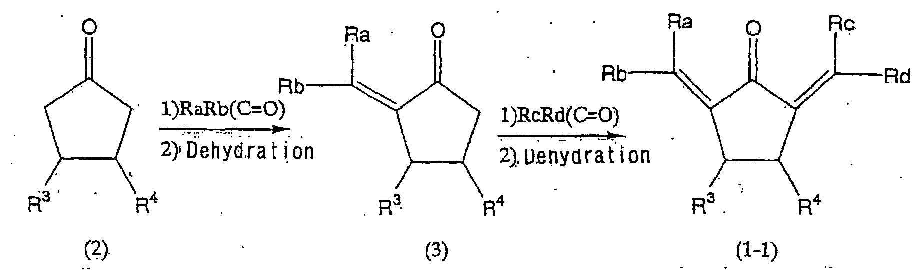 wherein R³ and R⁴ are as defined above, Ra and Rc independently represents an alkyl group having 1 to 6 carbon atoms, Rb and Rd independently represent a hydrogen atom or an alkyl group having 1 to 6 carbon atoms. Ra and Rb may be bonded together to form a cyclohexane ring, and Rc and Rd may be bonded together to form a cyclopentane ring or a cyclohexane ring, provided that the total number of carbon atoms in the sum of Ra and Rb is 3 to 6 and the total number of carbon atoms in the sum of Rc and Rd is 3 to 6.

The above-mentioned aldol reactions can be carried out in the presence of a base in a solvent. The base used includes, for example, metal alkoxides such as sodium methoxide, sodium ethoxide, potassium methoxide, potassium ethoxide and potassium t-butoxide; and metal hydroxides such as sodium hydroxide and potassium hydroxide. These bases may be used either alone or as a combination of at least two thereof.

The amount of base is usually in the range of 0.1 to 2 moles, preferably 0.5 to 1. 5 moles and more preferably 0.8 to 1.2 moles, per mole of the cyclopentanone compound (3-1).

The solvent used is not particularly limited, provided that it is an inert solvent, and, as specific examples thereof, there can be mentioned alcohols such as methanol and ethanol; aliphatic can be mentioned alcohols such as methanol and ethanol; aliphatic hydrocarbons such as n-hexane, n-heptane, n-octane, cyclopentane and cyclohexane; aromatic hydrocarbons such as benzene, toluene, ethylbenzene and xylene; and ethers such as diethyl ether, dibutyl ether, tetrahydrofuran and dioxane. These solvents may be used either alone or as a combination of at least two thereof.

The amount of aldehydes or ketones, represented by the formulae Ra-CO-Rb and Rc-CO-Rd is usually in the range of 0.1 to 2 moles, preferably 0.5 to 1.5 moles and more preferably 0.8 to 1.2 moles, per mole of the cyclopentanone compound (3-1).

The reaction temperature for the aldol reactions is usually in the range of 20 to 180°C, preferably 40 to 140°C and more preferably 60 to 100°C. The reaction can be carried out either under subatmospheric pressure or pressure.

The dehydration reactions are carried out by, for example, (i) a method wherein a reaction liquid containing an aldol reaction product is further heated as it is without isolation of the aldol reaction product; (ii) a method wherein a reaction liquid containing an aldol reaction product is neutralized, an acid catalyst is added to the neutralized reaction liquid, and then the reaction liquid is heated; or (iii) a method wherein an aldol reaction product is isolated from a reaction liquid containing the aldol reaction product, and the isolated reaction product is stirred in an appropriate solvent in the presence of an acid catalyst at a temperature in the range of room temperature to the boiling point of the solvent.

The acid catalyst used in the above-mentioned methods (ii) and (iii) includes, for example, inorganic acids such as hydrochloric acid and sulfuric acid; and organic acids such as paratoluenesulfonic acid, benzenesulfonic acid, methanesulfonic acid, acetic acid and oxalic acid. The amount of acid catalyst is usually in the range of 0.001 to 5 moles, preferably 0.01 to 3 moles, per mole of the aldol reaction product.

Among the cyclopentanone derivatives represented by the general formulae (1), (2) and (3), cyclopentanone derivatives (1-2) in which R¹ and R² are the same alkylidene group, R⁵ and R⁶ are the can be prepared by an aldol reaction between one molecule of cyclopentanone compounds (3-1) and two molecules of a compound represented by the formula Ra-CO-Rb, and a subsequent dehydrogenation reaction.

After completion of the dehydration reactions, the reaction products are subjected to conventional after-treatment and purification such as column chromatography, distillation or recrystallization whereby 2,5-dialkylidenecyclopentanone derivatives (1-1) and (1-2) can be isolated.

2,5-dialkylidenecyclopentanol derivative (1-3) can be prepared by reducing the carbonyl group of 2,5-dialkylidenecyclopentanone derivative (1-1) (see the following reaction scheme). 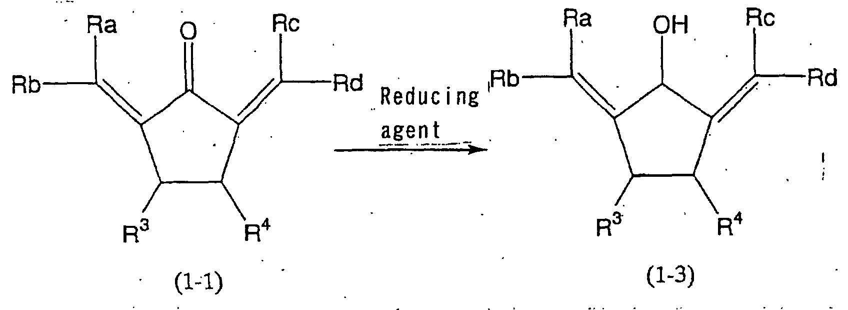 wherein R³, R⁴, Ra, Rb, Rc and Rd are the same as defined above.

2,5-dialkylcyclopentanol derivative (1-4) can be prepared by hydrogenating the carbon-carbon double bond of 2,5-dialkylidenecyclopentanone derivative (1-1) (see the following reaction scheme). 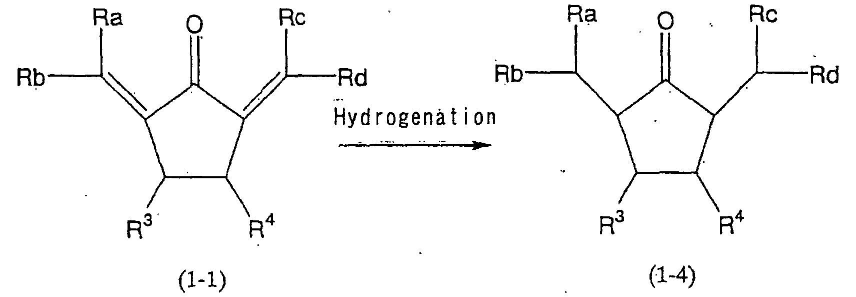 wherein R³, R⁴, Ra, Rb, Rc and Rd are the same as defined above.

2,5-dialkylcyclopentanol derivative (1-5) can be prepared by hydrogenating the carbon-carbon double bond of 2,5-dialkylidenecyclopentanol derivative (1-3). Alternatively, 2,5-dialkylcyclopentanol derivative (1-5) can be prepared by reducing the carbonyl group of 2,5-dialkylcyclopentanone derivative (1-4) (see the following reaction scheme). 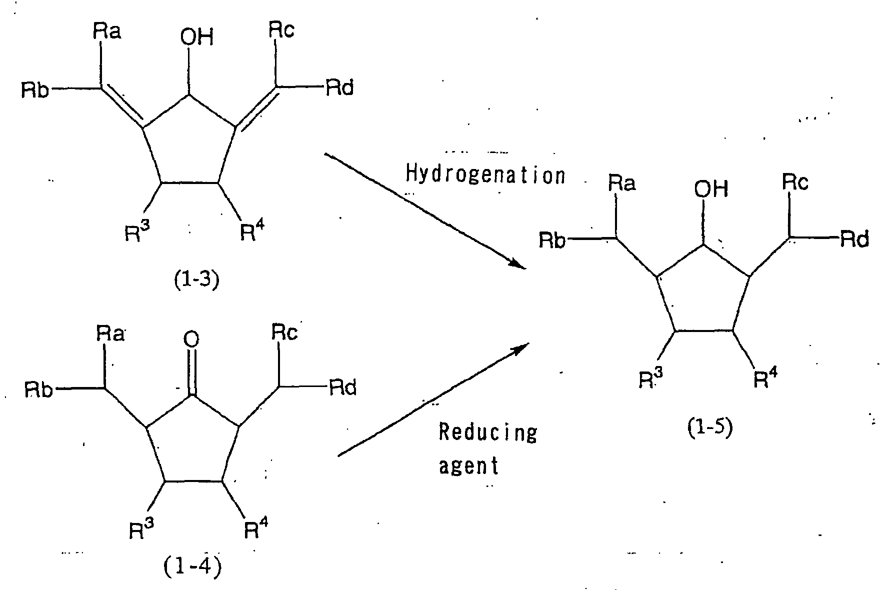 wherein R³, R⁴, Ra, Rb, Rc and Rd are the same as defined above.

The reducing procedures adopted for the above-mentioned reduction reactions are not particularly limited. In the case where the carbonyl group is reduced, a reducing agent such as sodium boron hydride (NaBH₄), lithium aluminum hydride (LiAlH₄) or diisobutylaluminum hydride (iBu₂AlH) is preferably used. In the case where the carbon-carbon double bond is hydrogenated, a catalytic hydrogenation reduction is preferably adopted using a hydrogenation catalyst such as palladium, ruthenium, rhodium, platinum or Raney nickel catalyst.

After completion of the reaction for obtaining cyclopentanone and cyclopentanol derivatives of the formulae (1-3) to (1-5), when a catalyst has been used for the reducing reaction, the catalyst is filtered off. When a reducing agent such as NaBH₄ or LiAlH₄ is used, the reaction liquid is neutralized with, for example, hydrochloric acid, and then, an organic phase is separated from an aqueous phase. Thereafter, the objective compound can be isolated by a conventional purifying procedure such as column chromatography, distillation or recrystallization.

Among the cyclopentanone derivatives of the present invention, a 2,5-dialkylcyclopentanone derivative of the formula (1-7) can also be prepared by the process shown in the following reaction scheme. 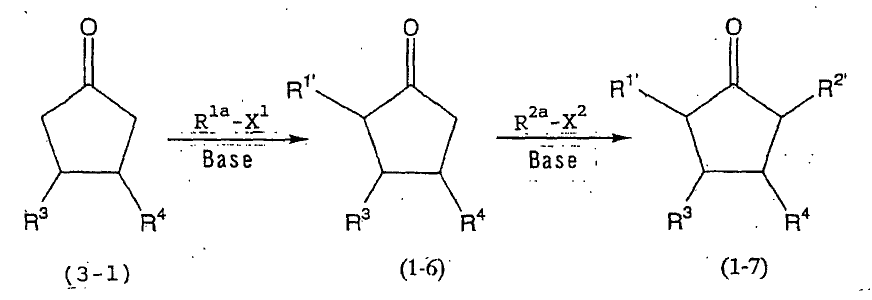 wherein R³ and R⁴ are as defined above, R^{1a} and R^{2a} independently represent an alkyl group having 4 to 7 carbon atoms, and X¹ and X² independently represent a halogen atom such as chlorine, bromine or iodine.

More specifically 2,5-dialkylcyclopentanone derivative (1-7) can be prepared by a process wherein cyclopentanone compound (3-1) is reacted with an alkyl halide represented by the formula R^{1a}-X¹ in the presence of a base in a solvent to give a monoalkylcyclopentanone derivative (1-6), and then, the monoalkylcyclopentanone derivative (1-6) is reacted with an alkyl halide represented by the formula R^{2a}-X² in the presence of a base in a solvent to give 2,5-dialkylcyclopentanone derivative (1-7).

As specific examples of the base used in the above reaction, there can be mentioned metal alkoxides such as sodium methoxide, sodium ethoxide, potassium methoxide, potassium ethoxide, potassium t-butoxide and magnesium ethoxide; metal hydrides such as sodium hydride, potassium hydride and calcium hydride; and organic bases such as 1,8-diazabicyclo[5.4.0]undec-7-ene (DBU) and 1,4-diazabicyclo[2.2.2]octane (Dabco).

As specific examples of the solvent used in the above reaction, there can be mentioned aromatic hydrocarbons such as benzene, toluene, xylene and ethylbenzene; aliphatic hydrocarbons such as n-hexane, n-heptane, n-octane, cyclopentane and cyclohexane; ethers such as diethyl ether, diisopropyl ether, dibutyl ether, tetrahydrofuran, dioxane and 1,2-dimethoxyethane; amides such as N,N-diemthylformamide, N,N-dimethylacetamide and N-methylpyrrolidone; and sulfur-containing compounds such as dimethylsulfoxide and sulfolane.

The amount of alkyl halides represented by the formulae R^{1a}-X¹ and R^{2a}-X², used in the above reactions, is usually in the range of 1 to 2 moles per mole of cyclopentanone compound (3-1). The amount of base used in the above reaction is usually in the range of 1 to 3 moles per mole of cyclopentanone compound (3-1). The above-mentioned reactions smoothly proceed at a temperature in the range of -20°C to the boiling of the solvent used. After completion of the reactions, the objective compound can be isolated by a conventional after-treatment and a purification procedure such as column chromatography, recrystallization or distillation.

A 2,5-dialkylcyclopentanol derivative of the formula (1-5) can also be prepared by a process wherein an aldol reaction of monoalkylcyclopentanone derivative (1-6) with an aldehyde or a ketone, represented by the formula: Rc-CO-Rd, and a subsequent dehydrogenation reaction are carried out to give a 2-alkyl-5-alkylidenecyclopentanone derivative (1-8), and then, the carbon-carbon double bond of the derivative (1-8) is hydrogenated and further the carbonyl group of the derivative (1-8) is reduced (see the following reaction scheme). 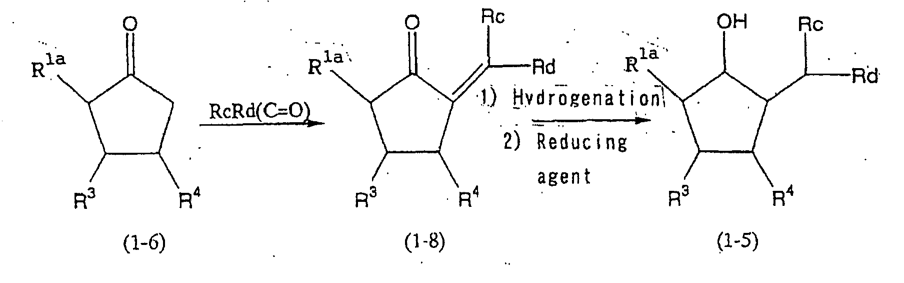 wherein R³, R⁴, R^{1a}, Rc and Rd are as defined above.

The starting material used in the above reaction, i.e., cyclopentanone compound (3-1), can be synthesized by a conventional process, for example, arprocess wherein cyclopentene is hydrated to give cyclopentanol, and the cyclopentanol is oxidized, or the starting material is commercially available.

Monoalkylcyclopentanone derivative (1-6) used in the above reaction can also be prepared by hydrogenating the carbon-carbon double bond of monoalkylidenecyclopentanone derivative (3-2).

The chemical structures of the above-mentioned compounds can be determined by NMR spectrum, IR spectrum, mass spectrum and elemental analysis.

The cyclopentanone derivatives of the present invention, represented by the formula (1), have a powdery and floral fragrance.

### (Examples)

The invention will now be described in more detail by the following examples that by no means limit the scope of the invention.

### Example 1 (Preparation of 2-n-pentyl-5-cyclopentylcyclopentanol)

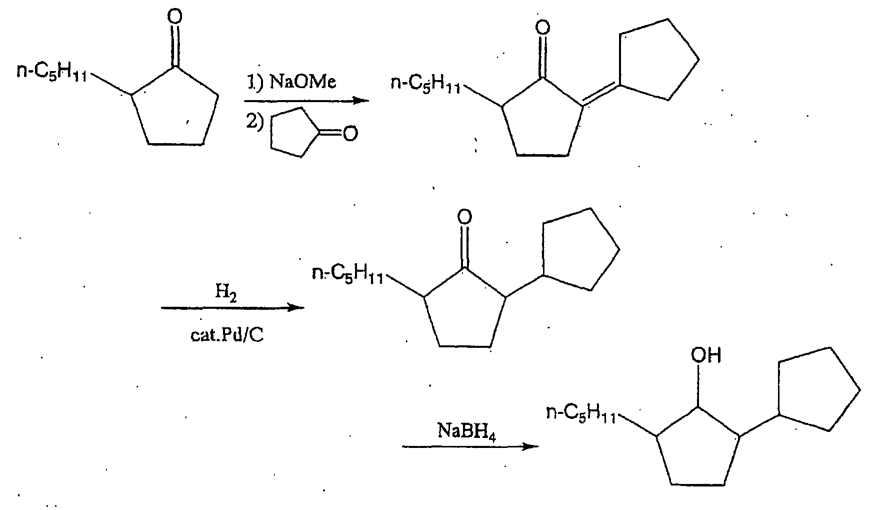

A four-necked three liter flask equipped with a stirrer, a reflux condenser, a dropping funnel and a thermometer was charged reflux condenser, a dropping funnel and a thermometer was charged with 482.5 g (2.5 moles) of 28% sodium methoxide (NaOMe) and 1,000 ml of ethylbenzene, and the temperature of the mixture was elevated to 80°C while being stirred. Thereafter 385.5 g (2.5 moles) of 2-n-pentylcyclopentanone was added over a period of 30 minutes, and the mixture was further stirred for 30 minutes. Then 210.5 g (2.5 moles) of cyclopentanone was added over a period of 20 minutes and the mixture was further stirred for 6 hours. To the reaction liquid, 1,000 g (2.75 moles) of 10% hydrochloric acid was added to neutralize the reaction liquid. An organic phase was separated from an aqueous phase, and dried over an anhydrous magnesium sulfate, and then filtered. The filtrate was concentrated, and the concentrate was purified by silica gel column chromatography using a mixed liquid of ethyl acetate/n-hexane (1:5) as an elute, to give 154 g of 2-n-pentyl-5-cyclopentylidenecyclopentanone. Yield: 28%.

Thereafter, a three-necked one liter flask equipped with a stirrer, a reflux condenser and a thermometer was charged with 154 g (0.7 mole) of 2-n-pentyl-5-cyclopentylidenecyclopentanone, 480 g of ethanol and 7.7 g of 5%Pd/C, and then flushed with nitrogen three times and then with hydrogen three times. Then the content was stirred at a temperature of 25 to 35°C and a pressure of 0 to 0.1 MPa for 8 hours. After completion of reaction, the catalyst was filtered off, and the filtrate was concentrated. Then the concentrate was purified by silica gel column chromatography using a mixed liquid of ethyl acetate/n-hexane (1:5) as an elute, to give 62.2 g of 2-n-pentyl-5-cyclopentylcyclopentanone with a purity of 99.5%. Yield: 40%.
¹H-NMR spectrum data (CDCl₃, TMS) δppm: 0.88 (end CH₃ of n-pentyl group at 2-position, 3H), 1.18-1.30, 1.34, 1.51-1.67, 1.75, 1.85, 1.90, 2.01-2.12, 2.18 (CH₂ of n-pentyl group at 2-position, CH₂ of cyclopentanone ring, and CH₂ of cyclopentyl group at 5-position, 20H), 1.96, 2.01-2.12 (CH of cyclopentanone ring, and CH of cyclopentyl group at 5-position, 3H)

Finally, a four-necked 300 ml flask equipped with a stirrer, a reflux condenser, a dropping funnel and a thermometer was charged with 9.6 g (0.25 mole) of sodium boron hydride and 100 ml of ethanol. While the content was stirred at a temperature of 30 to 40°C, 55.5 g (0.25 mole) of 2-n-pentyl-5-cyclopentylcyclopentanone was added over a period of 35 minutes, and the mixture was further stirred at the same temperature for 15 hours. Then the reaction liquid was poured into 380 g (0.4 mole) of 4% hydrochloric acid, and the reaction product was extracted with 150 ml of toluene. An organic phase was dried over an anhydrous magnesium sulfate, and then filtered. The filtrate was concentrated, and the concentrate was purified by silica gel column chromatography using a mixed liquid of ethyl acetate/n-hexane (1:5) as an elute, to give 35.3 g of 2-n-pentyl-5-cyclopentylcyclopentanol with a purity of 99%. Yield: 63%.

This compound had fruty, and fresh, floral green fragrance.
¹H-NMR spectrum data (CDCl₃, TMS) δppm: 0.84 (end CH₃ of n-pentyl group at 2-position, 3H), 1.15-1.50 (CH₂ of cyclopentanol ring, CH₂ of n-pentyl group at 2-position, and CH₂ of cyclopentyl group at 5-position, 20H), 1.63-1.90 (CH, 2H), 1.95 (-OH, 1H, this peak disappeared at treatment with D₂O), 3.85 (CH adjacent to OH group, 1H)
IR spectrum data (KBr): 3200-3550 cm⁻¹ (OH)

### Example 2 (Preparation of 2-cyclohexyl-5-cyclopentylcyclopentanol)

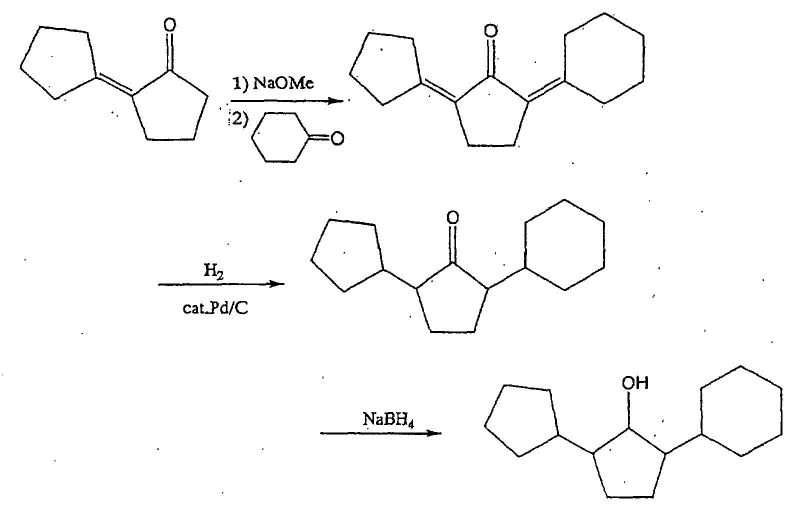

A four-necked one liter flask equipped with a stirrer, a reflux condenser, a dropping funnel and a thermometer was charged with 96.5 g (0.5 mole) of 28% sodium methoxide and 200 ml of ethylbenzene, and the temperature of the mixture was elevated to 80°C while being stirred. The content was further stirred for 30 minutes. Then 76.1 g (0.5 mole) of 2-cyclopentylideneclopentanone was added over a period of 20 minutes, and the mixture was further stirred for 30 minutes. Then 49.1 g (0.5 mole) of cyclohexanone was added over a period of 15 minutes and the mixture was further stirred for 5 hours. To the reaction liquid, 200 g (0.55 mole) of 10% hydrochloric acid was added to neutralize the reaction liquid. An organic phase was separated, and dried over an anhydrous magnesium sulfate, and then filtered. The filtrate was concentrated, and the concentrate was purified by silica gel column chromatography using a mixed liquid of ethyl acetate/n-hexane (1:5) as an elute, to give 25.7 g of 2-cyclohexylidene-5-cyclopentylidenecyclopentanone. Yield: 22%.

Thereafter, a three-necked one liter flask equipped with s stirrer, a reflux condenser and a thermometer was charged with 25. 7 g (0.11 mole) of 2-cyclohexylidene-5-cyclopentylidenecyclopentanone, 80 g of ethanol and 1.3 g of 5%Pd/C, and then flushed with nitrogen three times and then with hydrogen three times. Then the content was stirred at a temperature of 25 to 35°C and a pressure of 0 to 0.1 MPa for 10 hours. After completion of reaction, the catalyst was filtered off, and the filtrate was concentrated. Then the concentrate was purified by silica gel column chromatography using a mixed liquid of ethyl acetate/n-hexane (1:5) as an elute, to give 11.7 g of 2-cyclohexy-5-cyclopentylcyclopentanone with a purity of 99.3%. Yield: 45%.
¹H-NMR spectrum data (CDCl₃, TMS) δppm: 1.0, 1.15-1.28, 1.50-1.71, 1.85-1.92, 1.97-2.10 (CH₂ of cyclopentyl group at 2-position, CH₂ of cyclopentanone ring, and CH₂ of cyclohexyl group at 5-position, 22H), 1.97-2.1 (CH of cyclopentyl group at 2-position, CH of cyclopentanone ring, and CH of cyclohexyl group at 5-position, 4H)

Finally, a four-necked 300 ml flask equipped with a stirrer, a reflux condenser, a dropping funnel and a thermometer was charged with 1.9 g (0.05 mole) of sodium boron hydride and 120 ml of ethanol. While the content was stirred at a temperature of 30 to 40°C, 11.7 g (0.05 mole) of 2-cyclohexyl-5-cyclopentylcyclopentanone was added over a period of 35 minutes, and the mixture was further stirred for 18 hours. Then the reaction liquid was poured into 63 g (0.069 mole) of 4% hydrochloric acid, and the reaction product was extracted with 100 ml of toluene. An organic phase was dried over an anhydrous magnesium sulfate, and then filtered. The filtrate was concentrated, and the concentrate was purified by silica gel column chromatography using a mixed liquid of ethyl acetate/n-hexane (1:5) as an elute, to give 6.8 g of 2-cycclohexyl-5-cyclopentylcyclopentanol with a purity of 99.2%. Yield: 58%.

This compound had fresh, vanilla-like, sweat and herbal fragrance.
¹H-NMR spectrum data (CDCl₃, TMS) δppm: 1.02-1.25. 1.33-1.70, 1.80-1.95 (CH₂ of cyclopentyl group at 2-position, CH₂ of cyclopentanone ring, and CH₂ of cyclohexyl group at 5-position, 22H), 1.55, 1.87-1.95 (CH of cyclopentyl group at 2-position, CH of cyclopentanone ring, and CH of cyclohexyl group at 5-position, 3H), 1.93 (-OH, 1H, this peak disappeared at treatment with D₂O), 3.90 (CH adjacent to OH group, 1H)
IR spectrum data (KBr): 3200-3550 cm⁻¹ (OH)

### Example 3 (Preparation of 2,5-di-n-pentylcyclopentanol)

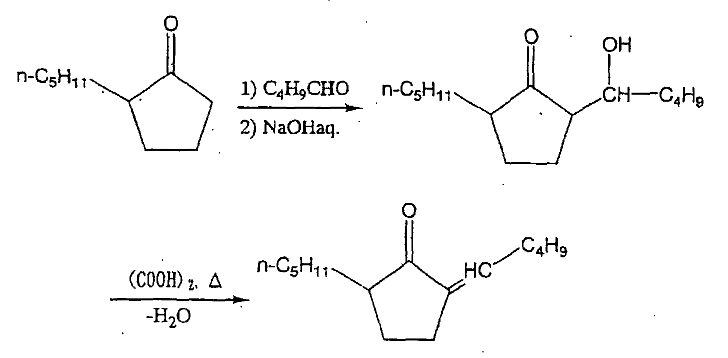

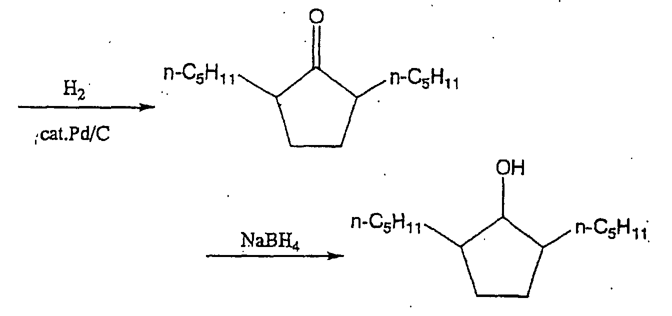

A four-necked 500 ml flask equipped with a stirrer, a thermometer, a nitrogen seal and a valeroaldehyde-dropping nozzle was charged with 138.6 g (0.9 mole) of 2-n-pentylcyclopentanone, 38 g of water and 2.2 g of an aqueous 25% by weight of sodium hydroxide solution. To the mixture, 43 g (0.5 mole) of valeroaldehyde was dropwise added at 25°C over a period of 2.5 hours, and the mixture was further stirred at the same temperature for 1 hour. To the reaction liquid, 57 g (0.015 mole) of 1% hydrochloric acid was added to neutralize the reaction liquid. An organic phase was separated, and dried over an anhydrous magnesium sulfate, and then filtered. Unreacted raw material was distilled off under reduced pressure from the filtrate, and the obtained residue was purified by silica gel column chromatography using a mixed liquid of ethyl acetate/n-hexane (1:5) as an elute,' to give 79.0 g of 2-(1-hydroxy)pentyl-5-n-pentylcyclopentanone. Yield: 37%.

Thereafter, a three-necked 500 ml flask equipped with s stirrer, a Dimroth condenser with a Dean-Stark trap, and a thermometer was charged with 79.0 g (0.33 mole) of 2-(1-hydroxy)pentyl-5-n-pentylcyclopentanone, 130 ml of toluene and 1.5 g (0.012 mole) of oxalic acid dehydrate. The content was refluxed for 3 hours, while water was removed from the content by the Dean-Stark trap. The reaction liquid was neutralized with 50 g of an aqueous 5% sodium bicarbonate solution. An organic phase was separated, and washed with water. Then, the washed organic phase was dried over anhydrous magnesium sulfate, and then filtered. The filtrate was concentrated, and then the concentrate was purified by silica gel column chromatography using a mixed liquid of ethyl acetate/n-hexane (1:5) as an elute, to give 31.5 g of 2-n-pentylidene-5-n-pentylcyclopentanone. Yield: 43%.

Thereafter, a three-necked 500 ml flask equipped with s stirrer, a reflux condenser and a thermometer was charged with 31.5 g (0.14 mole) of 2-n-pentylidene-5-n-pentylcyclopentanone, 100 g of ethanol and 1.6 g of 5%Pd/C, and then the content was flushed with nitrogen three times and then with hydrogen three times. Then the content was stirred at a temperature of 25 to 35°C and a pressure of 0 to 0.1 MPa for 7 hours. After completion of reaction, the catalyst was filtered off, and the filtrate was concentrated under reduced pressure. Then the concentrate was purified by silica gel column chromatography using a mixed liquid of ethyl acetate/n-hexane (1:5) as an elute, to give 14.9 g of 2,5-di-n-pentylcyclopentanone with a purity of 99.4%. Yield: 47%.
¹H-NMR spectrum data (CDCl₃, TMS) δppm: 0.88 (end CH₃ of cyclopentyl groups at 2- and 5-positions, 6H), 1.30, 1.60, 1.80, 2.20 (CH₂ of cyclopentyl groups at 2- and 5-positions, and CH₂ of cyclopentanone ring, 20H), 2.0 (CH of cyclopentanone ring, 2H)

Finally, a four-necked 300 ml flask equipped with a stirrer, a reflux condenser, a dropping funnel and a thermometer was charged with 2.5 g (0.066 mole) of sodium boron hydride and 120 ml of ethanol. While the content was stirred at a temperature of 30 to 40°C, 14.9 g (0.066 mole) of 2,5-di-n-pentylcyclopentanone was added over a period of 35 minutes, and the mixture was further stirred for 16 hours. Then the reaction liquid was poured into 84 g (0.092 mole) of 4% hydrochloric acid, and the reaction product was extracted with 100 ml of toluene. An organic phase was dried over an anhydrous magnesium sulfate, and then filtered. The filtrate was concentrated, and the concentrate was purified by silica gel column chromatography using a mixed liquid of ethyl acetate/n-hexane (1:5) as an elute, to give 9.0 g of 2,5-di-n-pentylcyclopentanol with a purity of 99.2%. Yield: 60%.

This compound had fresh, herbal-spicy and green fragrance.
¹H-NMR spectrum data (CDCl₃, TMS) δppm: 0.85 (end CH₃ of cyclopentyl groups at 2- and 5-positions, 6H), 1.2, 1.33, 1.76, 1.90 (CH₂ of cyclopentyl groups at 2- and 5-positions, and CH₂ of cyclopentanone ring, 20H), 1.7 (CH of cyclopentanone ring, 2H), 1.98 (-OH, 1H, this peak disappeared at treatment with D₂O), 3.75 (CH adjacent to OH group, 1H)
IR spectrum data (KBr): 3200-3550 cm⁻¹ (OH)

### Example 4 (Preparation of perfume composition)

2-n-pentyl-5-cyclopentylcyclopentanol prepared in Example 1 was mixed together with other perfume ingredients in amounts shown in Table 1 to prepare a perfume composition.

An organoleptic test of the perfume composition was carried out by seven panelists A, B, C, D, E, F and G. The test results are shown in Table 2.

**Table 1**

| Perfume ingredients | Amounts (parts by weight) |
|---|---|
| 2-n-pentyl-5-cyclopentylcyclopentan-1-ol | 10 |
| Linalyl acetate | 2 |
| Helional | 2 |
| Styralyl aceate | 4 |
| Methyl anthranilate | 4 |
| Dihydromyrcenol | 10 |
| Cis-3-hexenyl salicylate | 70 |
| Dimethylbenzylcarbinyl acetate | 10 |
| Terpineol | 10 |
| Benzyl acetate | 30 |
| LILIAL™ (Givaudin) | 40 |
| Phenylethyl alcohol | 50 |
| LYRAL™ (IFF) | 140 |
| CLAIGEON™ (Zeon Corporation) | 150 |
| Eugenol | 10 |
| Methyl ionon | 30 |
| Isocamphylcyclohexanol | 30 |
| Acetyl cedrene | 40 |
| GALAXOLIDE™ (IFF) | 60 |
| Diethyl phthalate | 258 |
| TONALID™ (PFW) | 40 |
| Total | 1,000 |
| Note, Name within parenthesis represents a manufacturing company. | |

**Table 2**

| Panelist | Evaluation results |
|---|---|
| A | Calm and exalted green fragrance |
| B | Green and sweet, and nobly and distinguishably dressed fragrance |
| C | Elegant floral, herbal and green fragrance |
| D | Mint gum-like fragrance |
| E | Exalted sweet and distinguishable fragrance |
| F | Jasmin-like sweet fragrance |
| G | Totally well balanced fragrance, and exalted floral green top-note |

### INDUSTRIAL APPLICABILITY

In accordance with the present invention, cyclopentanone derivatives useful as perfume ingredients having powdery and floral fragrance are provided. A perfume composition comprising the cyclopentanone derivative of the present invention emitting floral fragrance which is natural and fresh, and is useful for perfuming a variety of toiletries and households. In accordance with the present invention, a process for preparing the above-mentioned cyclopentanone derivatives is further provided by which the objective compounds can be obtained in a practically acceptable yield.

## Claims

1. A cyclopentanone derivative represented by the following general formula (1): 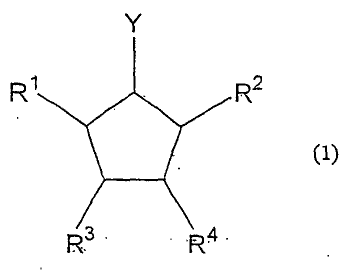 wherein R¹ represents an alkyl group having 4 to 7 carbon atoms, an alkylidene group having 4 to 7 carbon atoms, a cyclohexyl group or a cyclohexylidene group, R² represents an alkyl group having 4 to 7 carbon atoms, an alkylidene group having 4 to 7 carbon atoms; a cyclopentyl group, a cyclopentylidene group, a cyclohexyl group or a cyclohexylidene group, R³ and R⁴ independently represent a hydrogen atom or an alkyl group having 1 to 4 carbon atoms, and -Y represents -OH or =O.

2. A cyclopentanone derivative represented by the following general formula (2): 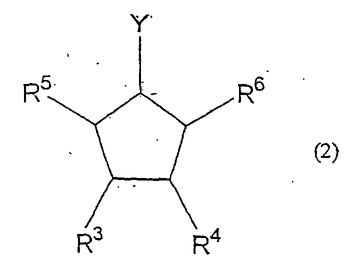 wherein R⁵ represents an alkyl group having 4 or 5 carbon atoms or an alkylidene group having 4 or 5 carbon atoms, R⁶ represents an alkyl group having 4 or 5 carbon atoms, an alkylidene group having 4 or 5 carbon atoms, a cyclopentyl group or a cyclopentylidene group, and R³, R⁴ and -Y are the same as defined above.

3. A cyclopentanone derivative represented by the following general formula (3): 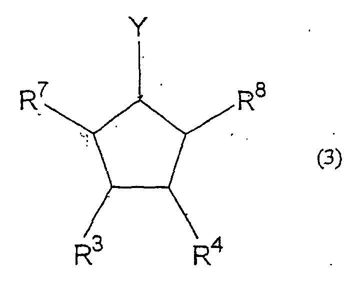 wherein R⁷ represents an alkyl group having 5 carbon atoms, or an alkylidene group having 5 carbon atoms, R⁸ represents an alkyl group having 5 carbon atoms, an alkylidene group having 5 carbon atoms, a cyclopentyl group, or a cyclopentylidene group, and R³, R⁴ and -Y are the same as defined above.

4. The cyclopentanone derivative according to any one of claims 1 to 3, which has 13 to 17 carbon atoms in total.

5. The cyclopentanone derivative according to any one of claims 1 to 4, wherein both of R³ and R⁴ are hydrogen atoms.

6. A perfume composition comprising at least one cyclopentanone derivative selected from those which are described in claims 1 to 5.

7. A perfume composition comprising 0.1 to 90% by weight of a cyclopentanone derivative selected from those which are described in claims 1 to 5.

8. A process for preparing a cyclopentanone derivative represented by the following general formula (6): 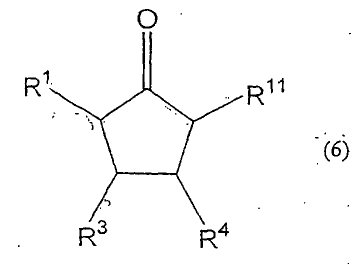 wherein R¹, R³ and R⁴ are the same as defined above, and R¹¹ represents an alkylidene group having 4 to 7 carbon atoms, a cyclopentylidene group or a cyclohexylidene group,
**characterized in that** a cyclopentanone derivative represented by the following general formula (4): 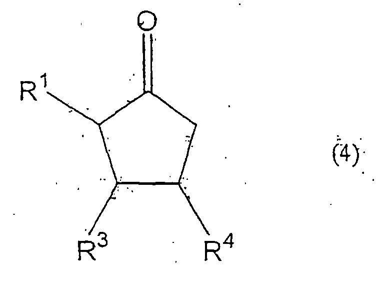 wherein R¹, R³ and R⁴ are the same as defined above, is reacted under alkaline conditions with a ketone or aldehyde represented by the following general formula (5): 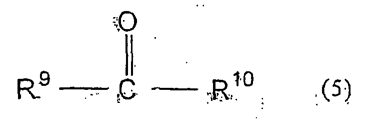 wherein R⁹ and R¹⁰ represents a hydrogen atom, or an alkyl group having 1 to 6 carbon atoms; the alkyl groups for R⁹ and R¹⁰ may be bonded together to form a ring; and the total number of carbon atoms in the sum of the alkyl groups for R⁹ and R¹⁰ is in the range of 3 to 6.

9. A process for preparing a cyclopentanone derivative represented by the following general formula (7): 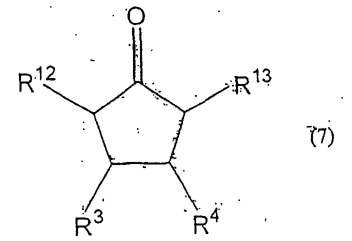 wherein R³ and R⁴ are the same as defined above, R¹² represents an alkyl group having 4 to 7 carbon atoms or a cyclohexyl group, and R¹³ represents an alkyl group having 4 to 7 carbon atoms, a cyclopentyl group or a cyclohexyl group,
**characterized in that** a cyclopentanone derivative represented by the above-mentioned general formula (6) is reduced with hydrogen.

10. A process for preparing a cyclopentanone derivative represented by the following general formula (8): 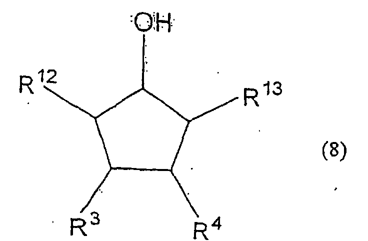 wherein R³, R⁴, R¹² and R¹³ are the same as defined above,
**characterized in that** a cyclopentanone derivative represented by the above-mentioned general formula (7) is reduced with hydrogen.

11. A process for preparing a cyclopentanone derivative represented by the above-mentioned general formula (8),
**characterized in that** a cyclopentanone derivative represented by the following general formula (9): 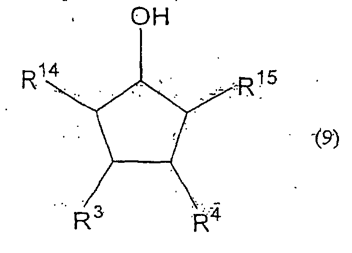 wherein R³ and R⁴ are the same as defined above, R¹⁴ represents an alkyl group having 4 to 7 carbon atoms, an alkylidene group having 4 to 7 carbon atoms, a cyclohexyl group or a cyclohexylidene group, R¹⁵ represents an alkyl group having 4 to 7 carbon atoms, an alkylidene group having 4 to 7 carbon atoms, a cyclopentyl group, a cyclopentylidene group, a cyclohexyl group or a cyclohexylidene group, and at least one of R¹⁴ and R¹⁵ is the alkylidene group, a cyclohexylidene group or a cyclopentylidene group, is reduced with hydrogen.
